(19) Europäisches Patentamt
European Patent Office
Office européen des brevets

(11) Publication number: 0 053 407 B1

(12) EUROPEAN PATENT SPECIFICATION

(45) Date of publication of patent specification: 30.12.86

(51) Int. Cl.$^4$: C 07 D 233/64, A 61 K 31/415

(21) Application number: 81201192.2

(22) Date of filing: 28.10.81

# (54) New imidazolylphenyl amidines, processes for their preparation and their pharmaceutical use, and intermediates of preparation.

(30) Priority: 28.11.80 IT 2632380

(43) Date of publication of application: 09.06.82 Bulletin 82/23

(45) Publication of the grant of the patent: 30.12.86 Bulletin 86/52

(84) Designated Contracting States: AT BE CH DE FR IT LI LU NL SE

(56) References cited:
US-A-4 069 340
US-A-4 182 910
US-A-4 236 015

(73) Proprietor: ISTITUTO DE ANGELI S.p.A.
Via Serio, 15
I-20139 Milano (IT)

(72) Inventor: Cereda, Enzo
Via Romagnolo 2A
I-15057 Tortona Alessandria (IT)
Inventor: Donetti, Arturo
Viale Romagna, 4
I-20133 Milano (IT)
Inventor: Del Soldato, Piero
Via E. Toti, 22
I-20052 Monza Milano (IT)
Inventor: Bergamaschi, Mario
Via Missori, 14
I-20052 Monza Milano (IT)

(74) Representative: Faraggiana, Vittorio et al
Ing. Barzanò & Zanardo S.P.A. Via Borgonuovo, 10
I-20121 Milano (IT)

EP 0 053 407 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to new pharmacologically active substituted imidazolylphenyl amidines which are $H_2$ receptor blocking agents, which inhibit gastric acid secretion and which are useful antiulcer agents.

The ineffectiveness of the ordinary antihistaminic drugs in blocking the stimulating effect of histamine on gastric secretion has prompted the search for histaminic antagonists involved in this response. This effect of histamine, which is recognized as a powerful agonist of gastric secretion is mediated through $H_2$ receptors (Black, *et al*, Nature *236*, 385, 1972) and is not inhibited by the classical antihistamines, $H_1$ receptor blockers (Ash and Schild, Brit. J. Pharmacol., *27*, 427, 1966). Investigations in this direction culminated in the synthesis of a class of substances (G. J. Durant, *et al*, J. Med. Chem. *20*, 901, 1977) typified by burimamide the first clinically effective $H_2$ receptor antagonist. Although burimamide was sufficiently selective pharmacologically it seemed to lack sufficient oral biovailability. Metiamide a subsequently evaluated $H_2$ antagonist, proved more potent than burimamide and orally active in man. However, metiamide could not be introduced into therapy owing to its toxic side effects (agranulocytosis).

Cimetidine, a congener of metiamide bearing a cyanoguanidino instead of a thioureido group, proved as potent as metiamide as a $H_2$ antagonist but devoid of the toxic side effects of metiamide.

Cimetidine has been recently introduced into therapy as an antiulcer drug. Its half-life however is relatively short and administration of several daily doses of 200—300 mg tablets is required. This shortcoming has prompted further research into the $H_2$ receptor blockers area aimed at finding longer acting and/or more potent substances. Recently, two new $H_2$— receptor antagonists [ranitidine (AH 19065) and tiotidine (ICI 125,225)] have been reported possessing chemical features analogous to cimetidine, i.e. a linear methylthioethyl side-chain bearing neutral polar groups. The main chemical variation in the latter compounds with respect to cimetidine consists in the replacement of the imidazole by an aminoalkyl furan and a 2-guanidinothiazole ring, respectively. Both ranitidine (Bradshaw, *et al*, Brit. J. Pharmacol. *66*, 464 P, 1979) and tiotidine (T. O. Yellin, Life Sci., *25*, 2001—9, 1979) have been reported to be more potent than cimetidine either as $H_2$ receptor antagonists in "in vitro" tests or as inhibitors of gastric acid secretion "in vivo".

We have now discovered that by substituting the linear side-chain, present in the classical as well as the two more recent $H_2$ receptor antagonists, with a phenyl ring possessing amidino groups optionally substituted, new substances are obtained endowed with potent $H_2$ — blocking activity, which inhibit the gastric acid secretion.

The $H_2$ receptor antagonists besides to be useful as blocking the gastric secretion agents are potentially useful as therapeutically active agents in the field of inflammation. [Yoshioka T., Monafo W. W, Ayvazian *et al.* (Am. J. Surg. 136,681 (1978)] and of cardiovascular disorders [Jerome P., Trzechiakowski and R. Levi (J. Pharmacol. Exp. Ther. *214*, 629—634, (1980)]. In some conditions it would be also useful a combination of $H_1$ and $H_2$ antagonists activity [Barry L. Tepperman, Eugene D. Jacobson *et al.* (Life Sciences, vol. 24, 2301—2308 (1979)]. Some compounds of the present invention show both these receptorial interations.

According to the present invention there are provided compounds of general formula I

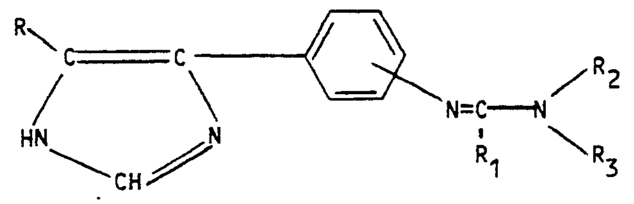

(I)

in which R, $R_1$ and $R_3$, which may be the same or different, represent a hydrogen atom or a lower alkyl group containing from 1 to 3 carbon atoms and $R_2$ represents a linear or branched alkyl, alkenyl or alkynyl group, a cyano group, a hydroxyl group, a substituted or unsubstituted cycloalkyl or cycloaliphatic alkyl group, a bicyclic group, an aralkyl or aryl group (optionally substituted by halogen, methyl, methoxy or methylenedioxy groups) or a substituted or unsubstituted heterocyclicalkyl or heterocyclic unsaturated five membered ring) which may also contain a further hetero atom; and non-toxic acid addition salts thereof.

The term "non-toxic" herein used designates salts formed with acids (either inorganic or organic) the anionic portions of which are physiologically compatible in the doses at which the salts are administered, salts formed with hydrochloric, maleic, fumaric, methansulfonic, citric, tartaric, acetic acid.

It is understood that for convenience in this specification reference will be made indifferently either to the compounds as bases or to the corresponding salts.

It is to be understood that although the double bond in the amidine radical and in the imidazole ring has been inserted in a particular position, various other tautomeric forms are possible, and the present invention includes such tautomeric forms within its scope, both in terms of the compounds of the inventions and in terms of the manufacturing processes.

When in the compounds of formula I R, $R_1$ and/or $R_3$ represents lower alkyl groups these may be, alkyl groups containing from 1 to 3 carbon atoms, e.g. methyl. When $R_2$ represents linear or branched alkyl

groups they may be alkyl groups containing from 1 to 8 carbon atoms; when $R_2$ is a linear or branched alkenyl groups these may be alkenyl groups containing from 2 to 5 carbon atoms; when $R_2$ represents linear or branched alkynyl groups they may be alkynyl groups from 3 to 4 carbon atoms; when $R_2$ is a bicyclic group it represents a norbornyl group; when $R_2$ represents an aralkyl group it is a benzyl group; when $R_2$ represents an aryl group it is a phenyl group (optionally substituted by a halogen atom, the halogen atom may, for example, be a chlorine atom), when $R_2$ represents substituted or unsubstituted cycloalkyl or cycloaliphatic alkyl groups they may contain from 3 to 6 carbon atoms; when $R_2$ represents a substituted or unsubstituted heterocylicalkyl or heterocyclic group it is an unsatured five-member ring which may also contain further heteroatom. In the above mentioned formula (I) the amidine radical may be in the ortho, meta or para position of the benzene ring.

Preferred compounds according to the present invention are those wherein the amidine radical is in the para position of the benzene ring and R, $R_1$ and $R_3$ are hydrogen atom and $R_2$ may be hydroxyl, methyl, ethyl, n-propyl, isopropyl, n-butyl, isobutyl, sec-butyl, t-butyl, neopentyl, hexyl, heptyl, octyl, allyl, α methylallyl, prenyl, propargyl, α methylpropargyl, cyano, norbornyl, benzyl, cyclopropyl, cyclopropylmethyl, dimethylcyclopropylmethyl, mentyl, cyclohexyl, cyclohexylmethyl, phenyl, p-chlorophenyl, p-methylphenyl, p-metoxyphenyl, methylenedioxyphenyl or 2-furylmethyl group and their non-toxic acid addition salts (as hereinbefore defined).

The compounds of general formula I may, for example, be prepared by the following processes which constitute object of the present invention too.

A. Reaction of an imidazolylphenylamine of Formula II

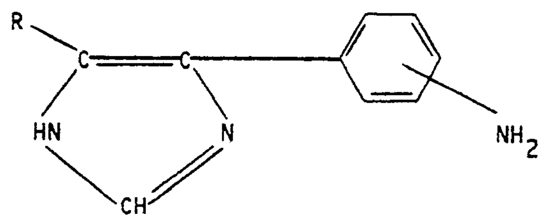

(II)

(wherein R is as hereinbefore defined) with a reactive derivative of a carboxamide of formula III

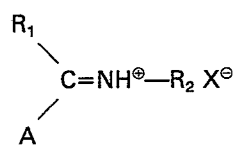

(III)

wherein $R_1$ and $R_2$ are as hereinbefore defined, $X^{\ominus}$ represents the anion of an inorganic acid such as hydrochloride or fluoroborate, and A represents a benzoyloxy group, chlorine or a lower alkoxy group such as methoxy or ethoxy. If desired the compound of formula III may be made to react also as a base. The reaction is generally effected at a temperature of from 0 to 100°C preferably from 20 to 60°C. The reaction is advantageously carried out in the presence of an inert organic solvent. Suitable solvents include for example alcohol having 1 to 3 carbon atoms such as methanol or ethanol, halogenated hydrocarbons such as dichloromethane, dioxane or acetone.

The compounds of formula III used as starting material in the above process may be obtained by conventional methods by reacting a carboximide of the formula IV

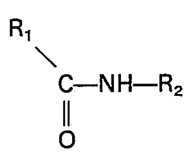

(IV)

wherein $R_1$ and $R_2$ are as above defined, with benzoylchloride, triethyloxomium fluoroborate, ethyl chloroformate, phosphorous oxychloride or phosphorous pentachloride.

B. Reaction of a N-(imidazolylphenyl)imidate of formula V

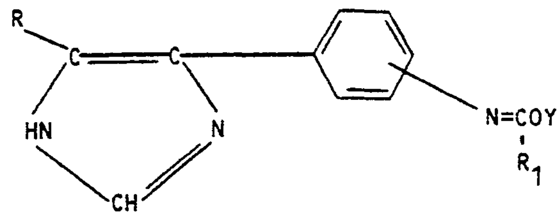

(V)

wherein R and $R_1$ are as hereinbefore defined and Y represents a lower alkyl group, such as methyl, ethyl, with an amine of formula VI

$$H_2N—R_2 \qquad (VI)$$

wherein $R_2$ is as hereinbefore defined.

The rection is conveniently carried out in the presence of an inert organic solvent. Suitable solvents include, for example alcohols such as methanol, ethanol, or dioxane.

The reaction may, however, be carried out in the absence of a solvent. An excess of amines of formula VI may be used as a solvent. The reaction is generally effected at a temperature of from 20 to 80°C.

If desired the above reaction may be carried out in a single step, by reacting amines of formula II with a compound of formula VII

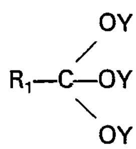

(VII)

wherein $R_1$ and Y are above defined, in the presence of an inorganic acid for example sulfuric acid, as catalyst. The reaction is carried out at a temperature between 60 to 120°C.

After a few hours the suitable amine of formula VI is added to the reaction mixture.

The compound of the general formula V used as starting material can be prepared by methods known in the literature, for example by reacting an amine of the formula II with a compound of the formula II with a compound of formula VII without solvent and distilling off the alcohol formed during the reaction.

C. Reaction of a N,N-disubstituted carboxamide dialkyl acetal of formula VIII

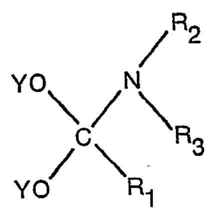

(VIII)

wherein $R_1$, $R_2$, $R_3$ and Y are as hereinbefore defined, with an amine of formula II. The reaction is performed at a temperature between 20 to 80°C and distilling the alcohol formed during the reaction.

D. Reaction of a N,N'-disubstituted amidine compound of formula IX

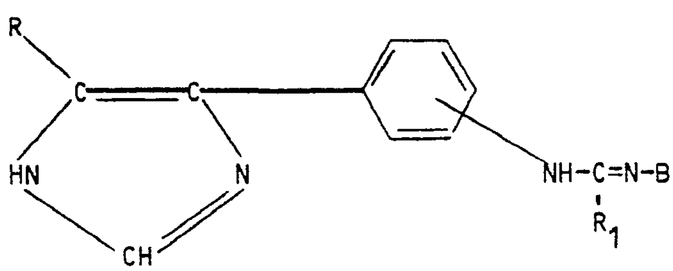

(IX)

wherein R and $R_1$ are as hereinbefore defined, B represents a cyano, acetyl, carbethoxy or carbamyl group with amines of formula (VI).

The reaction is conveniently performed in the presence of water or of an inert organic solvent for example alcohol such as methanol or ethanol, formamide, dioxane or acetonitrile. This reaction may for example be effected at a temperature of from 10—50°C preferably at room temperature.

The compounds of the formula IX used as starting material in the above process are obtained by processes that are known per se in the literature, for example, by reacting an amine of formula II with a N-substituted ethyl imidate of formula X

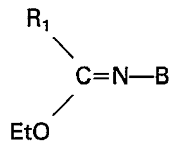

(X)

wherein $R_1$ and B are as hereinbefore defined, or optionally, when in the compound of formula IX B represents a cyano group, the reaction may also be carried out in a single step by reacting an amine of formula II with cyanamide in the presence of a compound of formula VII, in which Y and $R_1$ are as above defined.

The reaction is generally carried out in the presence of a suitable inert organic solvent for example a lower alcohol, ethers, ethylacetate, acetonitrile or dioxane or without solvent at a temperature of from 20 to 80°C. The compound of formula X may be prepared by conventional methods.

E. Reaction of a N,N'-disubstituted sulfinylamidine of formula XI

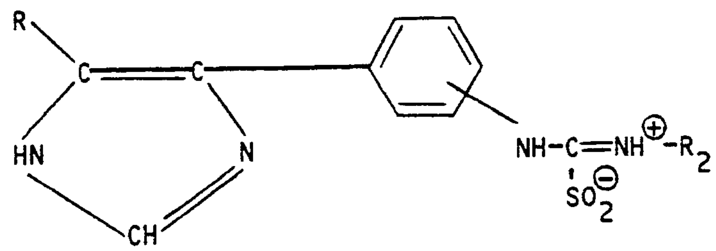

(XI)

where R, $R_2$ are as before defined, with a weak organic acid, for example formic, acetic or propionic acid.

N,N'-disubstituted sulfinylamidine of formula XI may be obtained by conventional methods for example by oxidizing the corresponding thiourea by means of hydrogen peroxyde in the presence of lower alcohol at temperatures of from 20—50°C.

F. Reaction in a single step of 1(N-alkiliminoformyl)-imidazole of formula XII

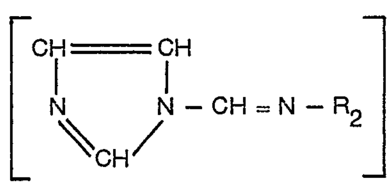

(XII)

(wherein $R_2$ is as hereinbefore defined) not isolated intermediate obtained in situ between an isonitrile of formula $C \equiv N-R_2$ and imidazole in the presence of AgCl according to T. Saegusa et al. [Tetrah. Letters 1283 (1974)] with an amine of formula II or directly by reacting an amine formula II with an isonitrile of formula $C \equiv N-R_2$ in the presence of CuCl. The reaction may be carried out without solvent or in the presence of an inert organic solvent, for example ethanol or dioxane.

The reaction is performed at temperature between 100—150°C.

The compounds of formula I prepared according to the processes A to F can then be converted with inorganic or organic acids into non-toxic acid addition salts as hereinbefore defined, for example by conventional method, such as by reacting the compounds as bases with a solution of the corresponding acids in a suitable solvent.

Particularly preferred acids include for example hydrochloric, maleic, fumaric, methanesulfonic acid. The salts obtained are normally soluble in water.

As above mentioned the new compounds of general formula I and their physiologically compatible acid addition salts are $H_2$ receptor blocking agents which inhibit the gastric acid secretion. Thus those compounds of general formula I wherein the amidine radical is in the para position of the benzene ring and R, $R_1$ and $R_3$ are hydrogen atoms and $R_2$ may be hydroxyl, methyl, ethyl, n-propyl, iso-propyl, n-butyl, isobutyl, sec-butyl, t-butyl, neopentyl, hexyl, heptyl, octyl, allyl, α-metylallyl, prenyl, propargyl, α-methyl-

propargyl, cyano, norbornyl, benzyl, cyclopropyl, cyclopropylmethyl, dimethylcyclopropylmethyl mentyl, cyclohexyl, cyclohexylmethyl, phenyl, p-chlorophenyl, p-methylphenyl, p-methoxyphenyl, methylenedioxyphenyl or 2-furylmethyl group and as well as their physiologically compatible acid addition salts have generally a better activity and therefore they are preferred compounds as antiulcer for the treatment of disorders of gastrointestinal tract.

Particularly preferred compounds according to the present invention are the following:

N-Ethyl-N'-[4-(imidazol-4-yl)-phenyl]-formamidine (Compound 11)
N-Isopropyl-N'-[4-(imidazol-4-yl)-phenyl]-formamidine (Compound 13)
N-Allyl-N'-[4-(imidazol-4-yl)-phenyl]-formamidine (Compound 14)
N-n Propyl-N'-[4-(imidazol-4-yl)-phenyl]-formamidine (Compound 18)
N-sec Butyl-N'-[4-(imidazol-4-yl)-phenyl]-formamidine (Compound 19)
N-Isobutyl-N'-[4-(imidazol-4-yl)-phenyl]-formamidine (Compound 20)
N-Prenyl-N-[4-(imidazol-4-yl)-phenyl]-formamidine (Compound 23)
N-Cyclopropylmethyl-N'-[4-(imidazol-4-yl)-phenyl]-formamidine (Compound 26)
N-Cyclopropyl-N'-[4-(imidazol-4-yl)-phenyl]-formamidine (Compound 32)
N-(α-Methylallyl)-N'-[4-(imidazol-4-yl)-phenyl]-formamidine (Compound 34)
N-Neopentyl-N'-[4-(imidazol-4-yl)-phenyl]-formamidine (Compound 36)
N-(2,2-Dimethyl)-cyclopropylmethyl-N'-[4-(imidazol-4-yl)-phenyl]-formamidine (Compound 37)

The antagonistic activity on histamine $H_2$ receptors is demonstrated either "in vitro" or "in vivo" by their inhibition of the $H_2$-dependent biological effects, which include the histamine evoked positive chronotropic effect and the histamine induced gastric secretion of acid respectively.

The positive chronotropic effect has been investigated in isolated guinea pig atria suspended in an organ bath (50 ml) containing oxygenated ($O_2$: 95% — $CO_2$: 5%) Krebs — Henseleit solution (pH 7.4) maintained at 32°C. The myocardial preparation loaded 1 g isometric tension, is allowed to stabilize 60 min., and myocardial contractions are recorded through an isometric lever connected to a strain-gauge coupler, and the instantaneous rate is monitored with a cardiotachymeter, and a heatwriting pen recorder. After control, responses (tachycardia) to histamine ($10^{-6}$ $^{g.ml^{-1}}$) are obtained three times at 30 minutes interval the test compounds are added to the bath to the desired final concentration, and the atria are again challenged with histamine. The chronotropic response obtained in the presence of the antagonist is then compared to the control response to histamine and the present reduction of the histamine $H_2$- evoked response is calculated. The average effective concentration ($EC_{50}$) of the $H_2$ antagonists is also calculatd by standard procedure according to D. R. Waud, Analysis of dose-response curves, in "Methods in Pharmacology" vol. 3 Smooth muscle, Ed Daniel E. E. Paton, M., Plenum Press. New York (1975); Ash and Schild, Br. J. Pharmacol. Chemother. *27*, 427—439, 1966.

The following Table I show the obtained results.

TABLE I

"In vitro" inhibitory activity in histamine induced tachycardia (guinea pig atria).

| Compound | $EC_{50}$ $10^{-8}$ M |
|---|---|
| 11 | 30 |
| 13 | 9.2 |
| 14 | 9.3 |
| 18 | 4.4 |
| 19 | 15.0 |
| 20 | 6.5 |
| 23 | 7.4 |
| 26 | 4.8 |
| 32 | 37.9 |
| 34 | 16.8 |
| 36 | 12.1 |
| 37 | 50 |
| CIMETIDINE | 340.0 |

Some compounds of formula 1 have been found to be effective to inhibit also the spasmodic action of histamine which is inhibited by classical $H_1$ antihistamines, such as diphenydramine and pyrilamine, in the isolated guinea-pig ileum.

The ability of the test compounds to inhibit histamine induced gastric secretion of acid, has been investigated after intravenous or intraduodenal administration in stomach perfused rats, according to Gosh and Shild (Br. J. Pharmacol. Chemother. 13, *54*, 1958).

The preparation of the animals in general anaesthesia (urethane, 1 g. $Kg^{-1}$ i.p.) and constant temperature, is achieved by inserting and tying in place polyethyl tubes (PE 50) in the aesophagus and in the pyloric-antral region. After the stomach is washed to remove residual of foods, continuous perfusion of the stomach was started with saline, 0.5 ml $min^{-1}$ (37°C), primed by a Jobling peristaltic pump. After 30 min of perfusion adaptation, the stomach perfusate is collected in 30 min samples, and titrated for acid content, expressed as µEq of NaOH 1 N. As control acid output becomes constant intravenous perfusion of histamine (1 mg. $kg^{-1}hr^{-1}$) is started and maintained throughout the experimental period. After the acid secretion has reached the steadily higher level, increasing doses of the test compounds are injected intravenously in order to obtain dose response functions. $ED_{50}$ were then calculated by standard procedure. The compounds tested by the aforementioned procedure showed a very potent antisecretory activity when administered intravenously at or below 100 µg/$kg^{-1}$, whose results are reported in the following table II:

TABLE II

"In vivo" antisecretory activity in histamine induced gastric secretion (stomach perfused rat).

| Compound | $ED_{50}$ mg/kg$^{-1}$ (i.v.)* |
|---|---|
| 11 | 0.02 |
| 13 | 0.0129 |
| 14 | 0.024 |
| 18 | 0.0111 |
| 19 | 0.0223 |
| 20 | 0.0259 |
| 23 | 0.0559 |
| 26 | 0.0235 |
| 32 | 0.0366 |
| 34 | 0.042 |
| 36 | 0.030 |
| 37 | 0.034 |
| CIMETIDINE | 0.560 |

* The values of activity are expressed taking the compound as a base.

The acute toxicity of the particularly preferred compounds of general formula I was approximately determined by oral administration of a single dose to groups of 5 Swiss mice fasting for 18 hours before the experiment. The evaluation of the incidence of mortality was considered after 14 days from the administration. The results are reported in the following table, in which the values are expressed taking the compound as a base.

TABLE III

| Compound | No. Dead/No. Treated Mice |
|---|---|
| 11 | 3/5 at 500 mg/kg |
| 13 | 2/5 at 500 mg/kg |
| 14 | 1/5 at 500 mg/kg |
| 18 | 2/5 at 375 mg/kg |
| 19 | 2/5 at 500 mg/kg |
| 20 | 2/5 at 250 mg/kg |
| 23 | 1/5 at 250 mg/kg |
| 26 | 4/5 at 500 mg/kg |
| 32 | 0/5 at 500 mg/kg |
| 34 | 2/5 at 500 mg/kg |
| 36 | 2/5 at 250 mg/kg |
| 37 | 5/5 at 250 mg/kg |

According to a further feature of the present invention there are provided pharmaceutical compositions comprising as active ingredient at least one compound of formula I, as hereinbefore defined, or a physiologically compatible acid addition salt thereof in association with a pharmaceutical carrier or excipient. For pharmaceutical administration the compound of general formula I and their physiologically compatible acid addition salts may be incorporated into the conventional pharmaceutical preparations in either solid or liquid form. The compositions may, for example, be presented in a form suitable for oral or parenteral administration. Preferred forms include for example, capsules, tablets, coated tablets, vials.

The active ingredient may be incorporated in excipients or carriers conventionally used in pharmaceutical compositions such as for example talc, gum arabic, lactose, gelatine, magnesium stearate, corn starch, aqueous or non-aqueous vehicles. The compositions are advantageously formulated as dosage unit, each dosage unit being adapted to supply a fixed dose of the active ingredient. Each dosage unit may conveniently contain 10 mg to 500 mg and preferably 20 mg to 150 mg.

The following examples illustrate some of the new compounds according to the present invention; these examples are not to be in any way considered limitative of the scope of the invention itself:

Example 1

*5-Methyl-4-(3-aminophenyl)-1-H-imidazole*

5-Methyl-4-(3-nitrophenyl)-1-H-imidazole [R. Morgenstern, *et al.* Pharmazie *30*, 103 (1975)] (84 g) dissolved in methanol was hydrogenated in the presence of Pd/C 5% (0.8 g) at atmospheric pressure and at room temperature. When the calculated amount of hydrogen was taken up, the catalyst was filtered off and the solution was evaporated to dryness to afford 62.5 g of the desired compound. M.p. 198—199°C.

Analysis:

| | | | | |
|---|---|---|---|---|
| $C_{10}H_{11}N_3$: | found % | C 69.12; | H 6.43; | N 24.40 |
| | calc. % | C 69.34; | H 6.40; | N 24.26 |

Example 2

(a) *5-Methyl-4-(4-nitrophenyl)-1-H-imidazole*

4-Methyl-4-phenyl-1-H-imidazole as a nitrate salt [R. Morgenstern, *et al.* Pharmazie *30*, 103 (1975)] (50 g) was added portionwise to 96% $H_2SO_4$ (100 ml). The thick solution so obtained was heated at 100°C for two hours, diluted with water (1000 ml) and again heated to 70°C for twenty hours. After cooling the solution was neutralized with 30% NaOH and 17% $Na_2CO_3$ aqueous solutions. The solid precipitated (a mixture of the *o*- and *p*-nitroderivative) was filtered and converted into its nitrate salt by means of 30% nitric acid. The mixture of salts of the two *o* and *p* nitro isomers so obtained was recrystallized from water to afford the pure para-isomer. Neutralization of an aqueous solution of the para-nitro derivative furnished

30 g of 5-methyl-4-(4-nitrophenyl)-1-H-imidazole as a base sufficiently pure to be used in the next step. M.p. 190—191°C.

Analysis:

| | | | | |
|---|---|---|---|---|
| $C_{10}H_9N_3O_2$: | found % | C 58.94; | H 4.42; | N 20.81 |
| | calc. % | C 59.10; | H 4.46; | N 20.68 |

(b) *5-Methyl-4-(4-aminophenyl)-1-H-imidazole*

5-Methyl-4-(4-nitrophenyl)-1-H-imidazole (60 g) dissolved in methanol was hydrogenated in the presence of Pd/c 5% (0.7 g) at atmospheric pressure and at room temperature. When the calculated amount of hydrogen was taken up, the catalyst was filtered off and the solution was evaporated to dryness to afford 43 g of 5-methyl-4-(4-aminophenyl)-1-H-imidazole, M.p. 215—216°C.

Analysis:

| | | | | |
|---|---|---|---|---|
| $C_{10}H_{11}N_3$: | found % | C 69.12; | H 6.29; | N 24.15 |
| | calc. % | C 69.34; | H 6.40; | N 24.26 |

## Example 3

(a) *N-[3-(Imidazol-4-yl)-phenyl]-ethyl acetimidate*

A mixture of 4-(3-aminophenyl)-1-H-imidazole (15.9 g) [W. Schunack, *et al*, Arch. Pharm. *306,* 934 (1973)] and triethyl orthoacetate (18.17 g) was heated until the calculated amount of ethanol was distilled. The resulting solid was recrystallized from petroleum ether-diethyl ether (1:1 v/v) to give 21.6 g of N-[3-(imidazol-4-yl)-phenyl]-ethyl acetimidate. M.p. 126—127°C.

Analysis:

| | | | | |
|---|---|---|---|---|
| $C_{13}H_{15}N_3O$: | found % | C 68.14; | H 6.64; | N 18.01 |
| | calc. % | C 68.10; | H 6.59; | N 18.33 |

The following intermediates were prepared in a similar manner:

— *N-[3-(5-Methylimidazol-4-yl)-phenyl]-ethyl acetimidate*

was obtained starting from 5-Methyl-4-(3-amino-phenyl)-1-H-imidazole and triethylorthoacetate. M.p. 130—131°C ($Et_2O$).

Analysis:

| | | | | |
|---|---|---|---|---|
| $C_{14}H_{17}N_3O$: | found % | C 69.32; | H 7.08; | N 17.15 |
| | calc. % | C 69.11; | H 7.04; | N 17.27 |

— *N-[4-(5-Methylimidazol-4-yl)-phenyl]-ethyl acetimidate*

was obtained starting from 5-Methyl-4-(4-amino-phenyl)-1-H-imidazole and triethylorthoacetate. M.p. 171—172°C ($Et_2O$).

Analysis:

| | | | | |
|---|---|---|---|---|
| $C_{14}H_{17}N_3O$ | Found % | C 69.43; | H 7.09; | N 17.34 |
| | calc. % | C 69.11; | H 7.04; | N 17.27 |

— *N-[4-Imidazol-4-yl)-phenyl]-ethyl acetimidate*

was obtained starting from 4-(4-amino-phenyl)-1-H-imidazole [E. Balaban et al. J. Chem. Soc. 2701 (1925)] and triethylorthoacetate. M.p. 144—147°C ($Et_2O$).

Analysis:

| | | | | |
|---|---|---|---|---|
| $C_{13}H_{15}N_3O$: | found % | C 68.21; | H 6.60; | N 18.14 |
| | calc. % | C 68.10; | H 6.59; | N 18.33 |

(b) *N-Methyl-N'-[4-(imidazol-4-yl)-phenyl]-acetamidine (Compound 1)*

To a solution of N-[4-(imidazol-4-yl)-phenyl] ethyl-acetimidate (8.02 g) in ethanol (20 ml) three portions of 33% methylamine in ethanol (8 g each; 24 g total) were added in a period of six days. The solution was evaporated to dryness and the residue was dissolved in 10% hydrochloric acid. The acid solution was washed with ethyl acetate and basified to pH 10 with 10% NaOH. The solid which separated was collected by filtration and dried to give the title compound (5.5 g). M.p. 226—227°C.

Analysis:

| | | | | |
|---|---|---|---|---|
| $C_{12}H_{14}N_4$: | found % | C 67.53; | H 6.53; | N 25.98 |
| | calc. % | C 67.26; | H 6.59; | N 26.15 |

Maleate salt. M.p. 170—171°C.

The following acetamidines were prepared in a similar manner, starting from the appropriate ethyl acetimidate as above described.

*N-Methyl-N'-[3-(5-methylimidazol-4-yl)-phenyl]-acetamidine (Compound 2)*

M.p. 201—202°C.

Analysis:

| | | | | |
|---|---|---|---|---|
| $C_{13}H_{16}N_4$: | found % | C 67.77; | H 7.05; | N 24.18 |
| | calc. % | C 68.39; | H 7.06; | N 24.54 |

Hydrochloride salt. M.p. 260—261°C.

*N-Methyl-N'-[3-(imidazol-4-yl)-phenyl]-acetamidine (Compound 3)*

M.p. 117—120°C.

Analysis:

| | | | | |
|---|---|---|---|---|
| $C_{12}H_{14}N_4$: | found % | C 66.86; | H 6.81; | N 25.82 |
| | calc. % | C 67.26; | H 6.59; | N 26.15 |

Hydrochloride salt. M.p. 288—290°C.

*N-Methyl-N'-[4-(5-Methylimidazol-4-yl)-phenyl]-acetamidine (Compound 4)*

M.p. 223—226°C.

Analysis:

| | | | | |
|---|---|---|---|---|
| $C_{13}H_{16}N_4$: | found % | C 68.45; | H 7.08; | N 24.36 |
| | calc. % | C 68.39; | H 7.06; | N 24.54 |

Hydrochloride salt. M.p. 283—285°C.

Example 4

*N-Methyl-N'-[4-(imidazol-4-yl)-phenyl]-formamidina (Compound 5)*

To a solution of N-methylformamide (2.36 g) in anhydrous diethyl ether (10 ml) was slowly added at 0°C a solution of benzoyl chloride (5.62 g) in anhydrous diethyl ether (30 ml). After one hour stirring, the white solid which formed was filtered and immediately added to a suspension of 4-(4-aminophenyl)-1-H-imidazole (1.59 g) in dioxane (50 ml). The mixture was stirred overnight and then evaporated to dryness. The residue was taken up in water and the solution was basified to pH 10 with 10% NaOH and the product which separated was extracted with ethylacetate. The organic solution was dried ($MgSO_4$) and evaporated to dryness to give 0.75 g of the crude amidine which was recrystallized from acetone. M.p. 189—190°C.

Analysis:

| | | | | |
|---|---|---|---|---|
| $C_{11}H_{12}N_4$: | found % | C 65.55; | H 5.92; | N 27.56 |
| | calc. % | C 65.98; | H 6.04; | N 27.98 |

Fumarate salt. M.p. 188—189°C.

Example 5

*N-Methyl-N'-[4-(imidazol-4-yl)-phenyl]-formamidine (Compound 5)*

To a solution of N-methylformamide (1.48 g) in 15 ml of dichloromethane, was slowly added at 0°C a solution of 4.78 g of triethyloxonium fluoroborate [H. Meerwein, Org. Synth. *46*, 113 (1966)] in dichloromethane (25 ml). After six hours stirring at room temperature, 4-(4-aminophenyl)-1-H-imidazole (2 g) in ethanol (10 ml) was added dropwise. The mixture was stirred overnight, and then evaporated to dryness. The fluoroborate salt of N-methyl-N'-[4-(imidazol-4-yl)-phenyl]-formamidine was dissolved in water and the solution was basified to pH 10 with 10% NaOH. The product which crystallized off was collected by filtration to give 1.4 g of the title formamidine. M.p. 189—190°C.

The following compounds were prepared in a similar manner, starting from the appropriate imidazolylphenylamine and from the suitable N-substituted formamide:

— *N-tert-Butyl-N'-[4-(imidazol-4-yl)-phenyl]-formamidine (Compound 21)*

Fumarate salt. M.p. 192—194°C (Ethanol).

Analysis:

| | | | | |
|---|---|---|---|---|
| $C_{22}H_{26}N_4O_8$: | found % | C 55.94; | H 5.67; | N 11.68 |
| | calc. % | C 55.69; | H 5.52; | N 11.81 |

— *N-Methyl-N'-[4-(imidazol-4-yl)-phenyl]-formamidine (Compound 38)*

Tartrate salt. M.p. 106—109°C.

Analysis:

| | | | | |
|---|---|---|---|---|
| $C_{28}H_{40}N_4O_{12}$: | found % | C 53.99; | H 6.48; | N 8.94 |
| | calc. % | C 53.84; | H 6.45; | N 8.97 |

Example 6

a) *N-Methyl-N'-[4-(imidazol-4-yl)-phenyl]-formamidine (Compound 5)*

A suspension of 4-(4-aminophenyl)-1-H-imidazole (0.8 g) and ethyl N-methyl-formimidate hydrochloride (1.86 g) [F. H. Suydam, et al., J. Org. Chem. *34*, 292 (1969)] in 20 ml of anhydrous ethanol was

stirred at room temperature for two days. The clear solution was evaporated to dryness, and the oily residue was dissolved in water. The solution so obtained was basified to pH 10 with 10% NaOH. The product which separated was extracted with ethyl acetate, the organic solution was evaporated to dryness and the product obtained was purified through its hydrochloride salt to give 0.35 g of the title compound as crystalline solid. M.p. 280—282°C (Ethanol).

Analysis:

| | | | | | |
|---|---|---|---|---|---|
| $C_{11}H_{14}Cl_2N_4$: | found % | C 47.98; | H 5.26; | Cl 25.78; | N 20.21 |
| | calc. % | C 48.36; | H 5.17; | Cl 25.96; | N 20.51 |

b) *N-Phenyl-N'-[4-(imidazol-4-yl)-phenyl]-formamidine (Compound 30)*

5 g of 4-(4-aminophenyl)-1-H-imidazole were added to a solution of ethyl N-phenyl-formimidate [Org. Synth., *35*, 65 (1955)] (4.6 g) in acetone (25 ml). After stirring for 4 hours at room temperature the solid separated was filtered and dried under vacuum. The product was treated with acetone and acidified with glacial acetic acid. N-Phenyl-N'-[4-(imidazol-4-yl)-phenyl]-formamidine crystallized as acetate salt which was filtered to give 4.6 g of the title product. M.p. 123—124°C.

Analysis:

| | | | | |
|---|---|---|---|---|
| $C_{20}H_{22}N_4O_4$: | found % | C 62.58; | H 5.72; | N 14.69 |
| | calc. % | C 62.81; | H 5.80; | N 14.65 |

The following compounds were prepared in a similar manner, starting from the appropriate imidazolylphenylamine and from the suitable ethyl formimidates:

— *N-3,4-Methylenedioxyphenyl-N'-[4-(imidazol-4-yl)-phenyl]-formamidine (Compound 39)*

Fumarate salt. M.p. 140—141°C.

Analysis:

| | | | | |
|---|---|---|---|---|
| $C_{25}H_{22}N_4O_{10}$: | found % | C 55.48; | H 4.15; | N 10.50 |
| | calc. % | C 55.76; | H 4.12; | N 10.41 |

— *N-4-Chlorophenyl-N'-[4-(imidazol-4-yl)-phenyl]-formamidine (Compound 41)*

Acetate salts. M.p. 78—78°C.

Analysis:

| | | | | | |
|---|---|---|---|---|---|
| $C_{20}H_{21}ClN_4O_4$: | found % | C 57.73; | H 5.12; | Cl 8.63; | N 13.36 |
| | calc. % | C 57.62; | H 5.08; | Cl 8.50; | N 13.44 |

— *N-4-Tolyl-N'-[4-(imidazol-4-yl)-phenyl]-formamidine (Compound 42)*

Acetate salt. M.p. 91—92°C.

Analysis:

| | | | | |
|---|---|---|---|---|
| $C_{21}H_{24}N_4O_4$: | found % | C 62.98; | H 6.13; | N 14.08 |
| | calc. % | C 63.62; | H 6.10; | N 14.13 |

— *N-4-Anisyl-N'-[4-(imidazol-4-yl)-phenyl]-formamidine (Compound 43)*

Fumarate salt. M.p. 131—132°C.

Analysis:

| | | | | |
|---|---|---|---|---|
| $C_{22}H_{24}N_4O_9$: | found % | C 57.01; | H 4.64; | N 10.71 |
| | calc. % | C 57.25; | H 4.61; | N 10.68 |

— *N-(3,4-Dimethylisoxazol-5-yl)-N'-[4-(imidazol-4-yl)-phenyl]-formamidine (Compound 44)*

M.p. 204—205°C.

Analysis:

| | | | | |
|---|---|---|---|---|
| $C_{15}H_{15}N_5O$: | found % | C 63.91; | H 5.40; | N 24.85 |
| | calc. % | C 64.04; | H 5.37; | N 24.90 |

## Example 7

(a) *N-Cyano-N'-[4-(imidazol-4-yl)-phenyl]-formamidine (Compound 6)*

A solution of 4-(4-aminophenyl)-1-H-imidazole (25.3 g) and ethyl N-cyano formimidate (18.7 g) [K. R. Huffman et al., J. Org. Chem. *28*, 1816, (1963)] in ethanol was stirred at room temperature overnight. The product which crystallized at the end of the reaction, was collected by filtration, washed with cold ethanol to give 30,7 g of the title compound. M.p. 234—235°C.

Analysis:

| | | | | |
|---|---|---|---|---|
| $C_{11}H_{19}N_5$: | found % | C 61.94; | H 4.36; | N 32.95 |
| | calc. % | C 62.55; | H 4.30; | N 33.16 |

The following compounds were prepared in a similar manner starting from the appropriate imidazolyl phenyl amine.

— *N-Cyano-N'-[3-(imidazol-4-yl)-phenyl]-formamidine (Compound 7)*
M.p. 206—207°C.
Analysis:

| | | | | |
|---|---|---|---|---|
| $C_{11}H_9N_5$: | found % | C 62.60; | H 4.22; | N 32.84 |
| | calc. % | C 62.55; | H 4.30; | N 33.16 |

— *N-Cyano-N'-[4-(5-methylimidazol-4-yl)-phenyl]-formamidine*
M.p. 236—238°C.
Analysis:

| | | | | |
|---|---|---|---|---|
| $C_{12}H_{11}N_5$: | found % | C 63.44; | H 5.04; | N 30.94 |
| | calc. % | C 63.98; | H 4.92; | N 31.09 |

— *N-Cyano-N'-[3-(5-methylimidazol-4-yl)-phenyl]-formamidine*
M.p. 221—223°C.
Analysis:

| | | | | |
|---|---|---|---|---|
| $C_{12}H_{11}N_5$: | found % | C 63.96; | H 5.06; | N 30.82 |
| | calc. % | C 63.98; | H 4.92; | N 31.09 |

(a') *N-Cyano-N'-[4-(imidazol-4-yl)-phenyl]-formamidine (Compound 6)*

A mixture of 4-(p-aminophenyl)-1-H-imidazole (113.8 g) ethyl ortoformiate (132.4 g) and cyanamide (30.7 g) was heated at 100°C for 20 minutes and therefore stirred at room temperature for 3 hours. The solid was filtered and dried to give 130 g of the title compound. M.p. 236—238°C.

(b) *N-Methyl-N'-[4-(imidazol-4-yl)-phenyl]-formamidine (Compound 5)*

N-Cyano-N'-[4-(imidazol-4-yl)-phenyl]-formamidine (20 g) was added in one portion to 35% methylamine in water (70 ml). After few minutes from the addition, the solid N-methyl-N'-[4-(imidazol-4-yl)-phenyl]-formamidine separated out of the solution. This solid was filtered, washed with water and dried to give 13.45 g of the title product, which was recrystallized from acetone. M.p. 189—190°C.

Fumarate salt. M.p. 188—189°C.

The following compounds were prepared in a similar manner, starting from the appropriate N-cyano formamide derivatives.

— *N-Methyl-N'-[3-(imidazol-4-yl)-phenyl]-formamidine (Compound 8)*
Maleate salt. M.p. 149—150°C (Ethanol).
Analysis:

| | | | | |
|---|---|---|---|---|
| $C_{19}H_{20}N_4O_8$: | found % | C 51.93; | H 4.73; | N 13.15 |
| | calc. % | C 52.78; | H 4.66; | N 12.96 |

— *N-Methyl-N'-[3-(5-methylimidazol-4-yl)-phenyl]-formamidine (Compound 9)*
M.p. 180—181°C.
Analysis:

| | | | | |
|---|---|---|---|---|
| $C_{12}H_{14}N_4$: | found % | C 67.40; | H 6.67; | N 26.40 |
| | calc. % | C 67.26; | H 6.59; | N 26.15 |

— *N-Methyl-N'-[4-(5-methylimidazol-4-yl)-phenyl]-formamidine (Compound 10)*
M.p. 225—226°C.
Analysis:

| | | | | |
|---|---|---|---|---|
| $C_{12}H_{14}N_4$: | found % | C 66.81; | H 6.70; | N 25.82 |
| | calc. % | C 67.26; | H 6.59; | N 26.15 |

— *N-Ethyl-N'-[4-(imidazol-4-yl)-phenyl]-formamidine (Compound 11)*
Hydrochloride salt. M.p. 252—254°C.
Analysis:

| | | | | | |
|---|---|---|---|---|---|
| $C_{12}H_{16}Cl_2N_4$: | found % | C 49.92; | H 5.70; | Cl 25.00; | N 19.56 |
| | calc. % | C 50.18; | H 5.61; | Cl 24.69; | N 19.51 |

— *N-Butyl-N'-[4-(imidazol-4-yl)-phenyl]-formamidine (Compound 12)*
Hydrochloride salt. M.p. 206—208°C.
Analysis:

| | | | | | |
|---|---|---|---|---|---|
| $C_{14}H_{20}Cl_2N_4$: | found % | C 53.48; | H 6.51; | Cl 22.63; | N 17.59 |
| | calc. % | C 53.34; | H 6.39; | Cl 22.49; | N 17.77 |

— *N-Isopropyl-N'-[4-(imidazol-4-yl)-phenyl]-formamidine (Compound 13)*
Fumarate salt. M.p. 130—133°C.

Analysis:
$C_{21}H_{24}N_4O_8$: found % C 53.91; H 5.30; N 12.36
calc. % C 54.78; H 5.25; N 12.17

— *N-Allyl-N'-[4-(imidazol-4-yl)-phenyl]-formamidine (Compound 14)*
Maleate salt. M.p. 153—154°C.
Analysis:
$C_{21}H_{22}N_4O_8$: found % C 54.85; H 4.71; N 11.93
calc. % C 55.02; H 4.84; N 12.22

— *N-Hydroxy-N'-[4-(imidazol-4-yl)-phenyl]-formamidine (Compound 15)*
Hydrochloride salt. M.p. 207—208°C.
Analysis:
$C_{10}H_{12}Cl_2N_4O$: found % C 43.70; H 4.34; Cl 25.48; N 20.45
calc. % C 43.65; H 4.39; Cl 25.76; N 20.36

— *N-n-Propyl-N'-[4-(imidazol-4-yl)-phenyl]-formamidine (Compound 18)*
Hydrochloride salt. M.p. 220—221°C (Ethanol).
Analysis:
$C_{13}H_{18}Cl_2N_3$: found % C 51.13; H 6.10; Cl 23.30; N 18.51
calc. % C 51.83; H 6.02; Cl 23.54; N 18.60

— *N-sec Butyl-N'-[4-(imidazol-4-yl)-phenyl]-formamidine (Compound 19)*
Hydrochloride salt. M.p. 196—197°C (Ethanol).
Analysis:
$C_{14}H_{20}Cl_2N_4$: found % C 52.97; H 6.51; Cl 22.08; N 17.44
calc. % C 53.33; H 6.39; Cl 22.49; N 17.77

— *N-Isobutyl-N'-[4-(imidazol-4-yl)-phenyl]-formamidine (Compound 20)*
Hydrochloride salt. M.p. 238—240°C (Ethanol).
Analysis:
$C_{14}H_{20}Cl_2N_4$: found % C 52.69; H 6.54; Cl 22.04; N 17.38
calc. % C 53.33; H 6.39; Cl 22.49; N 17.77

— *N-(1-Methyl)n hexyl-N'-[4-(imidazol-4-yl)-phenyl]-formamidine (Compound 22)*
Maleate salt. M.p. 150—151°C (Acetone).
Analysis:
$C_{25}H_{32}N_4O_8$: found % C 59.09; H 6.08; N 10.63
calc. % C 58.13; H 6.24; N 10.85

— *N-Prenyl-N'-[4-(imidazol-4-yl)-phenyl]-formamidine (Compound 23)*
Fumarate salt. M.p. 174—175°C (Ethanol).
Analysis:
$C_{23}H_{26}N_4O_8$: found % C 56.94; H 5.48; N 11.67
calc. % C 56.78; H 5.39; N 11.52

— *N-Propargyl-N'-[4-(imidazol-4-yl)-phenyl]-formamidine (Compound 24)*
Methanesulfonate salt. M.p. 184—185°C (Ethanol).
Analysis:
$C_{15}H_{20}N_4O_6S_2$: found % C 42.75; H 4.93; N 13.20; S 15.29
calc. % C 43.26; H 4.84; N 13.45; S 15.39

— *N-n Hexyl-N'-[4-(imidazol-4-yl)-phenyl]-formamidine (Compound 25)*
Hydrochloride salt. M.p. >270°C (Ethanol).
Analysis:
$C_{16}H_{24}Cl_2N_4$: found % C 55.35; H 7.00; Cl 20.39; N 16.00
calc. % C 55.97; H 7.05; Cl 20.66; N 16.32

— *N-Cyclopropylmethyl-N'-[4-(imidazol-4-yl)-phenyl]-formamidine (Compound 26)*
Fumarate salt. M.p. 127—128°C.
Analysis:
$C_{22}H_{24}N_4O_8$: found % C 56.21; H 5.09; N 11.99
calc. % C 55.93; H 5.12; N 11.86

— *N-Bicyclo-[2,2,1]-Hept-2-yl-N'-[4-(imidazol-4-yl)-phenyl]-formamidine (Compound 27)*
Maleate salt. M.p. 182—183°C.
Analysis:

| | | | | |
|---|---|---|---|---|
| $C_{25}H_{28}N_4O_8$: | found % | C 57.90; | H 5.43; | N 10.94 |
| | calc. % | C 58.58; | H 5.51; | N 10.93 |

— *N-Furfuryl-N'-[4-(imidazol-4-yl)-phenyl]-formamidine (Compound 28)*
Hydrochloride salt. M.p. 215—217°C (Ethanol).
Analysis:

| | | | | | |
|---|---|---|---|---|---|
| $C_{15}H_{16}Cl_2N_4O$: | found % | C 52.80; | H 4.87; | Cl 20.62; | N 16.27 |
| | calc. % | C 53.11; | H 4.75; | Cl 20.90; | N 16.52 |

— *N-Cyclohexyl-N'-[4-(imidazol-4-yl)-phenyl]-formamidine (Compound 29)*
Fumarate salt. M.p. 139—141°C (Ethanol).
Analysis:

| | | | | |
|---|---|---|---|---|
| $C_{24}H_{28}N_4O_8$: | found % | C 57.89; | H 5.68; | N 10.99 |
| | calc. % | C 57.59; | H 5.64; | N 11.20 |

— *N-Benzyl-N'-[4-(imidazol-4-yl)-phenyl]-formamidine (Compound 31)*
Hydrochloride salt. M.p. 210—212°C (Ethanol).
Analysis:

| | | | | | |
|---|---|---|---|---|---|
| $C_{17}H_{18}Cl_2N_4$: | found % | C 57.91; | H 5.29; | Cl 19.94; | N 15.87 |
| | calc. % | C 58.46; | H 5.19; | Cl 20.30; | N 16.04 |

— *N-Cyclopropyl-N'-[4-(imidazol-4-yl)-phenyl]-formamidine (Compound 32)*
Fumarate salt. M.p. 185—186°C (Ethanol).
Analysis:

| | | | | |
|---|---|---|---|---|
| $C_{21}H_{22}N_4O_8$: | found % | C 55.09; | H 4.74; | N 12.48 |
| | calc. % | C 55.02; | H 4.84; | N 12.22 |

— *N-(α-Methylpropargyl)-N'-[4-(imidazol-4-yl)-phenyl]-formamidine (Compound 33)*
Maleate salt. M.p. 188°C.
Analysis:

| | | | | |
|---|---|---|---|---|
| $C_{22}H_{22}N_4O_8$: | found % | C 56.28; | H 4.15; | N 11.87 |
| | calc. % | C 56.16; | H 4.17; | N 11.91 |

— *N-(α-Methylallyl)-N'-[4-(imidazol-4-yl)-phenyl]-formamidine (Compound 34)*
Maleate salt. M.p. 186°C.
Analysis:

| | | | | |
|---|---|---|---|---|
| $C_{22}H_{22}N_4O_8$: | found % | C 55.87; | H 5.04; | N 11.76 |
| | calc. % | C 55.93; | H 5.12; | N 11.86 |

— *N-n Octyl-N'-[4-(imidazol-4-yl)-phenyl]-formamidine (Compound 35)*
Fumarate salt. M.p. 152—153°C.
Analysis:

| | | | | |
|---|---|---|---|---|
| $C_{26}H_{34}N_4O_8$: | found % | C 58.52; | H 6.35; | N 10.72 |
| | calc. % | C 58.85; | H 6.46; | N 10.56 |

— *N-Neopentyl-N'-[4-(imidazol-4-yl)-phenyl]-formamidine (Compound 36)*
Fumarate salt. M.p. 251—252°C.
Analysis:

| | | | | |
|---|---|---|---|---|
| $C_{23}H_{28}N_4O_8$: | found % | C 56.67; | H 5.81; | N 11.40 |
| | calc. % | C 56.55; | H 5.78; | N 11.47 |

— *N-(2,2-Dimethyl)-cyclopropylmethyl-N'-[4-(imidazol-4-yl)-phenyl]-formamidine (Compound 37)*
Hydrochloride salt. M.p. 246—248°C.
Analysis:

| | | | | | |
|---|---|---|---|---|---|
| $C_{16}H_{22}Cl_2N_4$: | found % | C 55.90; | H 6.53; | Cl 20.88; | N 16.30 |
| | calc. % | C 56.30; | H 6.50; | Cl 20.78; | N 16.42 |

— *N-Cyclohexylmethyl-N'-[4-(imidazol-4-yl)-phenyl]-formamidine (Compound 40)*
Fumarate salt. M.p. 154—155°C.
Analysis:

| | | | | |
|---|---|---|---|---|
| $C_{25}H_{30}N_4O_8$: | found % | C 57.79; | H 5.91; | N 10.75 |
| | calc. % | C 58.36; | H 5.88; | N 10.89 |

Example 8

— *N,N-Dimethyl-N'-[4-(imidazol-4-yl)-phenyl]-formamidine (Compound 16)*

A solution of 4-(4-aminophenyl)-1-H-imidazole (3.18 g) in N,N-dimethyl-formamide diethylacetal (8.82 g) was stirred at room temperature for two days. Addition of diethyl ether afforded precipitation of the formamidine (Compound 16) which was collected by filtration (3.9 g). M.p 141—142°C.

Analysis:

| | | | | |
|---|---|---|---|---|
| $C_{12}H_{14}N_4$: | found % | C 67.02; | H 6.50; | N 26.28 |
| | calc. % | C 67.26; | H 6.59; | N 26.15 |

— *N-N,N-Dimethyl-N'-[4-(5-methylimidazol-4-yl)-phenyl]-formamidine (Compound 17)*

was prepared in a similar manner starting from 5-Methyl-4-(4-aminophenyl)-1-H-imidazole. M.p. 228—229°C.

Analysis:

| | | | | |
|---|---|---|---|---|
| $C_{13}H_{16}N_4$: | found % | C 69.08; | H 7.16; | N 24.28 |
| | calc. % | C 68.39; | H 7.06; | N 24.54 |

Example 9

*N-Methyl-N'-[4-(imidazol-4-yl)-phenyl]-formamidine (Compound 5)*

A mixture of 4-(4-aminophenyl)-1-H-imidazole (1.59 g) and triethyl orthoformate (2.22 g) was heated for 30 min. at 100°C in the presence of catalytic amounts of 96% $H_2SO_4$. A solution of methylamine 33% in ethanol (9.5 g) was then added dropwise to the cooled reaction mixture. The solution was stirred at room temperature for twenty hours and then evaporated to dryness. The residue obtained was dissolved in 10% hydrochloric acid and the acid solution was washed with ethyl acetate and then basified to pH 10 with 10% NaOH. The product which separated was extracted with ethyl acetate to give, after evaporation of the solvent, 0.45 g of the compound 5. M.p. 188—189°C.

Example 10

(a) *N-Methyl-N'-[4-(imidazol-4-yl)-phenyl]-thiourea*

A solution of 4-(4-aminophenyl)-1-H-imidazole (2.3 g) and methyl isothiocyanate (1.15 g) in 30 ml of ethanol was refluxed for 0.5 hour and evaporated to dryness. The oily base so obtained was purified by conversion into the corresponding hydrochloride salt by bubbling gaseous hydrochloric acid in isopropanol. The desired thiourea was obtained as a white solid (2 g) M.p. 210—211°C.

Analysis:

| | | | | | | |
|---|---|---|---|---|---|---|
| $C_{11}H_{13}ClN_3S$: | found % | C 49.23; | H 4.98; | Cl 13.20; | N 20.64; | S 11.72 |
| | calc. % | C 49.15; | H 4.87; | Cl 13.19; | N 20.84; | S 11.92 |

(b) *N-Methyl-N'-[4-(imidazol-4-yl)-phenyl]-formamidine sulfinic acid*

Hydrogen peroxide (31%, 1.08 g) was added dropwise in 30 min. to stirred ice-cooled solution of N-methyl-N'-[4-(imidazol-4-yl)-phenyl]-thiourea (1.16 g) in 5 ml of methanol. After two hours stirring the mixture was evaporated to dryness and the residue so obtained was crystallized from ethanol to afford 0.95 g of the desired compound.

Analysis:

| | | | | | |
|---|---|---|---|---|---|
| $C_{15}H_{12}N_4O_2S$: | found % | C 50.17; | H 4.55; | N 21.41; | S 12.04 |
| | calc. % | C 49.98; | H 4.57; | N 21.20; | S 12.12 |

Acetate salt. M.p. 194—195°C (Acetone).

(c) *N-Methyl-N'-[4-(imidazol-4-yl)-phenyl]-formamidine (Compound 5)*

N-Methyl-N'-[4-(imidazol-4-yl)-phenyl]-formamidine sulfinic acid (0.9 g) was dissolved in 15 ml of glacial acetic acid and refluxed for one hour. The concentrated solution was basified to pH 10 with 10% NaOH and extracted with ethyl acetate. The organic solution was evaporated to dryness to afford 0.3 g of N-methyl-N'-[4-(imidazol-4-yl)-phenyl]-formamidine. M.p. 187—189°C.

Example 11

— *N-t. Butyl-N'-[4-(imidazol-4-yl)-phenyl]-formamidine (Compound 21)*

A mixture of 4-(p-aminophenyl)-1-H-imidazole (2.3 g), t-butylisonitrile (1.25 g), AgCl (0.2 g) and imidazole (0.11 g) was heated at 100°C for 13 hours. The thick reaction mixture was treated with 10% HCl and ethyl acetate. The acid solution was taken to pH 6.5 with 10% NaOH, treated with charcoal, filtered and completely basified. The layer which separates was extracted into ethyl acetate. After drying the organic solution was evaporated to give 0.38 g of the title compound.

Fumarate salt. M.p. 192—194°C.

Example 12

— *N-Cyclohexyl-N'-[4-(imidazol-4-yl)-phenyl]-formamidine (Compound 29)*

A mixture of 4-(p-aminophenyl)-1-imidazole (1.59 g) cyclohexyl isonitrile (1.2 g) and CuCl (0.99 g) was

heated at 150°C for 3 hours. The thick reaction mixture was extracted into methanol. The methanol solution after treated with charcoal was evaporated to dryness and extracted into ethyl acetate and 10% hydrochloric acid. The acid solution was basified to PH 7.5 with 17% $Na_2CO_3$ and extracted into ethyl acetate. The organic solution was evaporated to dryness. The residue after recrystallization from acetone give 0.25 g of the title product. M.p. 220—221°C.

Fumarate salt. M.p. 139—141°C.

The following not limitative examples of pharmaceutical compositions according to the invention are reported.

Example 13

| Tablets | |
|---|---|
| — active ingredient | 50 mg |
| — lactose | 217 mg |
| — corn starch | 30 mg |
| — magnesium stearate | 3 mg |

Example 14

| Capsules | |
|---|---|
| — active ingredient | 50 mg |
| — corn starch | 170 mg |
| — magnesium stearate | 2 mg |

Example 15

| Vials | |
|---|---|
| — active ingredient | 50 mg |
| — water for injection q.s. | 5 ml |

**Claims**

1. Compounds of general formula I

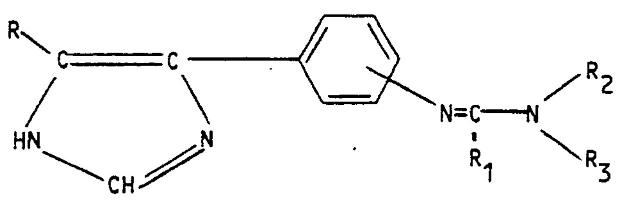

(I)

wherein R, $R_1$ and $R_3$, which may be the same or different, represent a hydrogen atom or a lower alkyl group containing from 1 to 3 carbon atoms and $R_2$ represents a linear or branched alkyl, alkenyl or alkynyl group, a cyano group, a hydroxyl group, a substituted or unsubstituted cycloalkyl or cycloaliphatic alkyl group, a bicyclic group, an aralkyl or aryl group (optionally substituted by halogen, methyl, methoxy or methylenedioxy groups), or a substituted or unsubstituted heterocyclic alkyl or heterocyclic unsaturated five membered ring which may also contain a further heteroatom.

2. Non-toxic acid addition salts of the compounds of general formula I as claimed in claim 1.

3. Salts as claimed in claim 2 formed with hydrochloric, fumaric, methanesulfonic or maleic acid.

4. Compounds as claimed in claim 1 wherein R, $R_1$ and $R_3$ represent a hydrogen atom.

5. Compounds as claimed in claims 1 to 4 wherein the amidine radical is in the para position of the benzene ring.

6. Compounds as claimed in claims 1, 4 and 5 wherein $R_2$ represents hydroxyl, methyl, ethyl, n-propyl, isopropyl, n-butyl, isobutyl, sec-butyl, t-butyl, neopentyl, hexyl, heptyl, octyl, allyl, α-metylallyl, prenyl, propargyl, α-methylpropargyl, cyano, norbornyl, benzyl, cyclopropyl, cyclopropylmethyl, dimethylcyclopropylmethyl, mentyl, cyclohexyl, cyclohexylmethyl, phenyl, p-chlorophenyl, p-methylphenyl, p-methoxyphenyl, methylenedioxyphenyl or 2-furylmethyl group.

7. Non-toxic acid addition salts of the compounds as claimed in claim 6.

8. Salts as claimed in claim 7 formed with hydrochloric, fumaric, methanesulfonic or maleic acid.

9. N-Ethyl-N'-[4-(imidazol-4-yl)-phenyl]-formamidine.

10. N-Isopropyl-N'-[4-(imidazol-4-yl)-phenyl]-formamidine.

11. N-Allyl-N'-[4-(imidazol-4-yl)-phenyl]-formamidine.

12. N-n-Propyl-N'-[4-(imidazol-4-yl)-phenyl]-formamidine.

13. N-sec Butyl-N'-[4-(imidazol-4-yl)-phenyl]-formamidine.

14. N-Isobutyl-N'-[4-(imidazol-4-yl)-phenyl]-formamidine.

15. N-Prenyl-N'-[4-(imidazol-4-yl)-phenyl]-formamidine.

16. N-Cyclopropylmethyl-N'-[4-(imidazol-4-yl)-phenyl]-formamidine.

17. N-Cyclopropyl-N'-[4-(imidazol-4-yl)-phenyl]-formamidine.

18. N-(α-Methylallyl)-N'-[4-(imidazol-4-yl)-phenyl]-formamidine.

19. N-Neopentyl-N'-[4-(imidazol-4-yl)-phenyl]-formamidine.

20. N-(2,2-Dimethyl)-cyclopropylmethyl-N'-[4-(imidazol-4-yl)-phenyl]-formamidine.

21. Compounds as claimed in any of claims 9 to 20 in the form of their hydrochloric, fumaric, methanesulfonic or maleic acid addition salts.

22. A process for the preparation of compounds of general formula I as defined in claim 1 or claim 2 which comprises reacting a compound of formula II

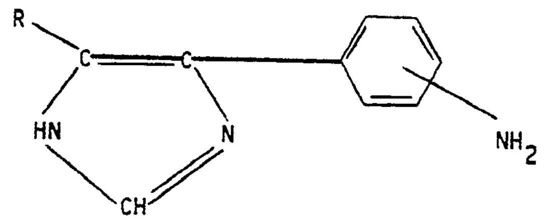

(II)

(wherein R is as defined in claim 1) with a compound of formula III)

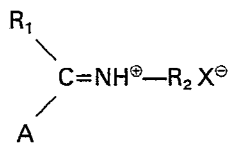

(III)

in which $R_1$ and $R_2$ are as defined in claim 1, $X^{\ominus}$ represents hydrochloride or fluoroborate and A represents a benzoyloxy group, chlorine or ethoxy group.

23. A process as claimed in claim 22 in which a compound of formula III is made to react as a base.

24. A process as claimed in claims 22 and 23 in which the reaction is carried out in the presence of an inert organic solvent.

25. A process as claimed in claim 24 in which the inert organic solvent comprises ethanol, dioxane or acetone.

26. A process as claimed in claims 22 to 25 in which the reaction is carried out at a temperature of from 20 to 60°C.

27. A process for the preparation of compounds of general formula I as defined in claim 1 or claim 2, which comprises reacting a compound of formula V

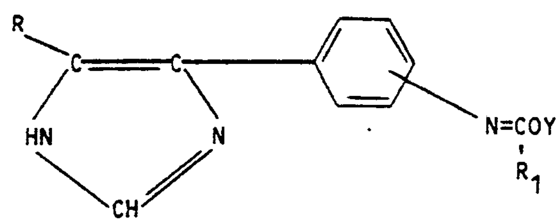

(V)

wherein R and $R_1$ are as defined in claim 1 and Y represents methyl or ethyl group with an amine of the formula VI

$$H_2N—R_2 \qquad (VI)$$

where $R_2$ is as defined in claim 1.

28. A process as claimed in claim 27 in which the reaction is carried out in an inert organic solvent.

29. A process as claimed in claim 28 in which the inert organic solvent is ethanol.

30. A process as claimed in claims 27 to 29 in which the reaction temperature is from 20—80°C.

31. A process as claimed in claims 27 in which an amine of formula II, a compound of formula VII

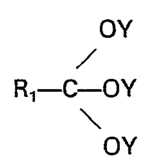

(VII)

(where $R_1$ and Y are as defined in claim 1 and claim 27) and an amine of formula VI are reacted in a single step in the presence of a catalyst.

32. A process as claimed in claim 31 in which the catalyst is sulfuric acid.

33. A process as claimed in claims 31 and 32 in which the reaction is carried out at a temperature of from 60 to 120°C.

34. A process for the preparation of compounds of general formula I as claimed in claim 1 or claim 2 which comprises reacting a formula VIII

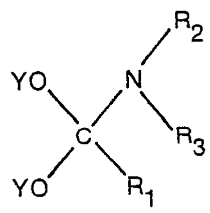

(VIII)

wherein $R_1$, $R_2$, and $R_3$ and Y are as defined in claim 1 and claim 27 with an amine of formula II.

35. A process as claimed in claim 34 in which the reaction is carried out at a temperature of from 20 to 80°C.

36. A process for the preparation of compounds of general formula I as claimed in claim 1 or claim 2 which comprises reacting a compound of formula IX

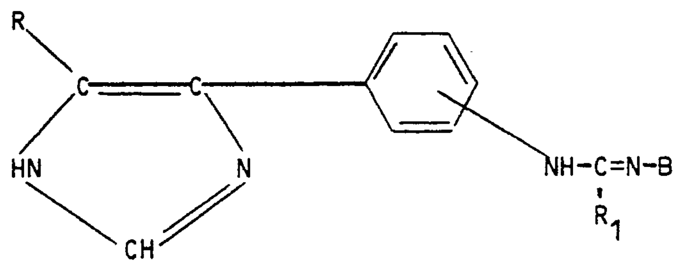

(IX)

wherein R and $R_1$ are as defined in claim 1, B represents a cyano, acetyl, carboethoxy or carbamyl group, with an amine of formula VI.

37. A process as claimed in claim 36 in which the reaction is carried out in presence of water.

38. A process as claimed in claims 36 and 37 in which the reaction is effected at room temperature.

39. A process for the preparation of compounds of general formula I as claimed in claim 1 or claim 2 which comprises reacting a compound of formula XI

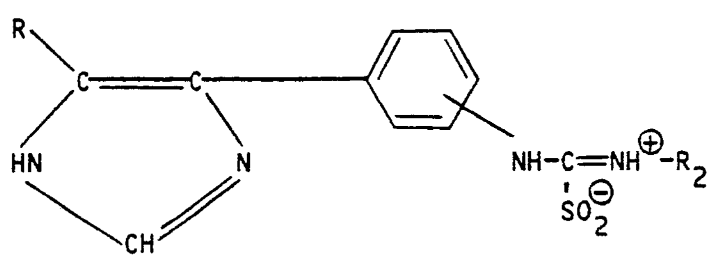

(XI)

wherein R and $R_2$ are as defined in claim 1, with a weak organic acid.

40. A process as claimed in claim 39 in which the weak organic acid comprises formic, acetic or propionic acid.

41. A process for the preparation of compounds of general formula I as claimed in claim 1 or claim 2 which comprises reacting a compound, not isolated obtained in situ between isonitrile of formula $C \equiv N—R_2$ and imidazole in presence of AgCl of formula XII

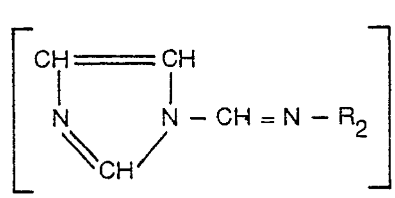

(XII)

wherein $R_2$ is as defined in claim 1, with an amine of formula II.

42. A process as claimed in claim 41, in which an amine of formula II is directly reacted with isonitrile of formula $C \Leftarrow N—R_2$ in the presence of CuCl.

43. A process as claimed in claims 41 and 42 in which the reaction is carried out without solvent or in the presence of an inert organic solvent.

44. A process as claimed in claim 43, in which the inert organic solvent comprises ethanol, dioxane.

45. A process as claimed in claims 41 to 44 in which the reaction temperature is between 100—150°C.

46. Intermediate compounds of formula

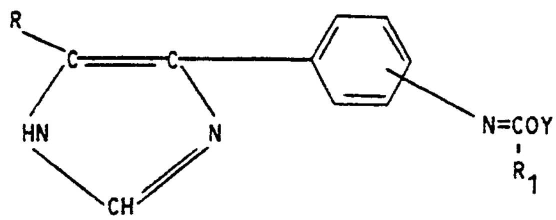

(V)

in which R, $R_1$ and Y are as defined in claims 1 and 27 for the performance of the process as claimed in claim 27.

47. Intermediate compounds of formula

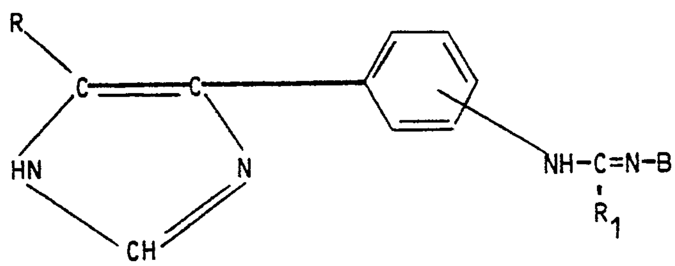

(IX)

wherein R, $R_1$ and B are as defined in claim 1 and claim 36, for the performance of the process as claimed in claim 36.

48. Intermediate compounds of formula

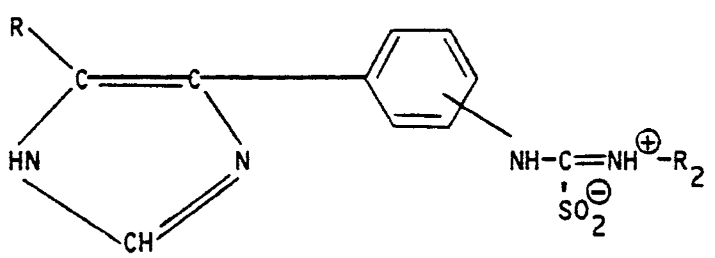

(XI)

wherein R and $R_2$ are as defined in claim 1, for the performance of the process as claimed in claim 39.

49. Pharmaceutical compositions comprising as active ingredient at least one compound as claimed in claim 1 or claim 2 in association with usual pharmaceutical carriers or excipients.

50. Pharmaceutical composition as claimed in claim 50 for use as anti-ulcers.

51. Pharmaceutical compositions as claimed in claim 50 for treatment of patients suffering from disorders of gastrointestinal tract.

**Patentansprüche**

1. Verbindungen der allgemeinen Formel I

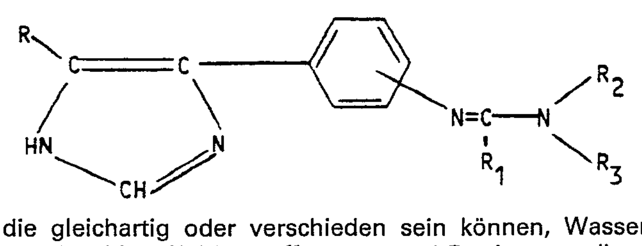

(I)

in der R, $R_1$ und $R_3$, die gleichartig oder verschieden sein können, Wasserstoffatome oder niedrigmolekulare Alkylgruppen mit 1 bis 3 Kohlenstoffatomen und $R_2$ eine geradkettig oder verzweigte Alkyl-,

Alkenyl- oder Alkinyl-Gruppe, eine Cyanogruppe, eine Hydroxylgruppe, eine substituierte oder unsubstituierte Cycloalkyl- oder cycloaliphatische Alkyl-Gruppe, eine bicyclische Gruppe, eine Aralkyl- oder Aryl-Gruppe (die gegebenenfalls durch Halogen, Methyl-, Methoxy- oder Methylendioxy-Gruppen substituiert ist) oder eine substituierte oder unsubstituierte heterocyclische Alkyl-Gruppe oder ein heterocyclischer ungesättigter fünfgliedriger Ring, der auch ein zusätzliches Heteroatom enthalten kann, bedeuten.

2. Nicht-toxische Säureadditionssalze der Verbindungen der Formel I, wie sie in Anspruch 1 beansprucht sind.

3. Salze gemäß Anspruch 2, die mit Chlorwasserstoffsäure, Fumarsäure, Methansulfonsäure oder Maleinsäure gebildet sind.

4. Verbindungen nach Anspruch 1, in der R, $R_1$ und $R_3$ jeweils Wasserstoffatome bedeuten.

5. Verbindungen nach den Ansprüchen 1 und 4, in denen das Amidinradikal in der para-Stellung des Benzolringes steht.

6. Verbindungen nach den Ansprüchen 1, 4 und 5, in denen $R_2$ eine Hydroxylgruppe, eine Methylgruppe, eine Ethylgruppe, eine n-Propylgruppe, eine Isopropylgruppe, eine n-Butylgruppe, eine Isobutylgruppe, eine sec-Butylgruppe, eine tert-Butylgruppe, eine Neopentylgruppe, eine Hexylgruppe, eine Heptylgruppe, eine Octylgruppe, eine Allylgruppe, eine α-Methylallylgruppe, eine Prenylgruppe, eine Propargylgruppe, eine α-Methylpropargylgruppe, eine Cyanogruppe, eine Norbornylgruppe, eine Benzylgruppe, eine Cyclopropylgruppe, eine Cyclopropylmethylgruppe, eine Dimethylcyclopropylmethylgruppe, eine Menthylgruppe, eine Cyclohexylgruppe, eine Cyclohexylmethylgruppe, eine Phenylgruppe, eine p-Chlorphenylgruppe, eine p-Methylphenylgruppe, eine p-Methoxyphenylgruppe, eine Methylendioxyphenylgruppe oder eine 2-Furylmethylgruppe bedeutet.

7. Nicht-toxische Säureadditionssalze der Verbindungen, die in Anspruch 6 beansprucht sind.

8. Salze nach Anspruch 7, die mit Chlorwasserstoffsäure, Fumarsäure, Methansulfonsäure oder Maleinsäure gebildet sind.

9. N-Ethyl-N'-[4-(imidazol-4-yl)-phenyl]-formamidin.

10. N-Isopropyl-N'-[4-(imidazol-4-yl)-phenyl]-formamidin.

11. N-Allyl-N'-[4-(imidazol-4-yl)-phenyl]-formamidin.

12. N-n-Propyl-N'-[4-(imidazol-4-yl)-phenyl]-formamidin.

13. N-sec.-Butyl-N'-[4-(imidazol-4-yl)-phenyl]-formamidin.

14. N-Isobutyl-N'-[4-(imidazol-4-yl)-phenyl]-formamidin.

15. N-Prenyl-N'-[4-(imidazol-4-yl)-phenyl]-formamidin.

16. N-Cyclopropylmethyl-N'-[4-(imidazol-4-yl)-phenyl]-formamidin.

17. N-Cyclopropyl-N'-[4-(imidazol-4-yl)-phenyl]-formamidin.

18. N-(α-Methylallyl)-N'-[4-(imidazol-4-yl)-phenyl]-formamidin.

19. N-Neopentyl-N'-[4-(imidazol-4-yl)-phenyl]-formamidin.

20. N-(2,2-Dimethyl)-cyclopropylmethyl-N'-[4-(imidazol-4-yl)-phenyl]-formamidin.

21. Verbindungen nach einem der Ansprüche 9 bis 20 in Form der Säureadditionssalze mit Chlorwasserstoffsäure, Fumarsäure, Methansulfonsäure oder Maleinsäure.

22. Verfahren zur Herstellung der Verbindungen mit der allgemeinen Formel I, wie sie in den Ansprüchen 1 oder 2 definiert sind, dadurch gekennzeichnet, daß man eine Verbindung der Formel II

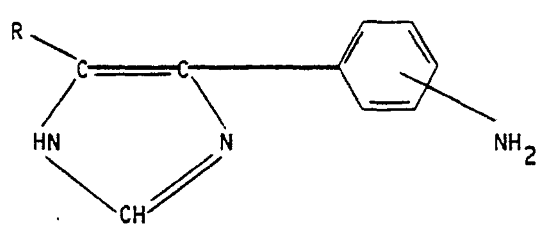

(II)

(worin R die in Anspruch 1 angegebenen Bedeutungen besitzt) mit einer Verbindung der Formel II

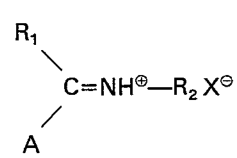

(III)

in der $R_1$ und $R_2$ die in Anspruch 1 angegebenen Bedeutungen besitzen, $X^{\ominus}$ für eine Hydrochlorid oder ein Fluoroborat steht und A eine Benzoyloxygruppe, ein Chloratom oder eine Ethoxygruppe darstellt, umsetzt.

23. Verfahren nach Anspruch 22, dadurch gekennzeichnet, daß man die Verbindung der Formel III als eine Base reagieren läßt.

24. Verfahren nach Ansprüchen 22 und 23, dadurch gekennzeichnet, daß man die Reaktion in Gegenwart eines inerten organischen Lösungsmittels durchführt.

25. Verfahren nach Anspruch 24, dadurch gekennzeichnet, daß man als inertes organisches Lösungsmittel Ethanol, Dioxan oder Aceton einsetzt.

26. Verfahren nach den Ansprüchen 22 bis 25, dadurch gekennzeichnet, daß man die Reaktion bei einer Temperatur von 20 bis 60°C durchführt.

27. Verfahren zur Herstellung der Verbindungen der allgemeinen Formel I, wie sie in Anspruch 1 oder Anspruch 2 definiert sind, dadurch gekennzeichnet, daß man eine Verbindung der Formel V

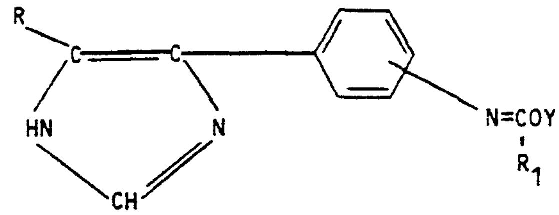

(V)

in der R und $R_1$ die in Anspruch 1 angegebenen Bedeutungen besitzen und Y eine Methylgruppe oder eine Ethylgruppe darstellt, mit einem Amin der Formel VI

$$H_2N—R_2 \qquad (VI)$$

in der $R_2$ die in Anspruch 1 angegebenen Bedeutungen besitzt umsetzt.

28. Verfahren nach Anspruch 27, dadurch gekennzeichnet, daß man die Reaktion in einem inerten organischen Lösungsmittel durchführt.

29. Verfahren nach Anspruch 28, dadurch gekennzeichnet, daß man als inertes organisches Lösungsmittel Ethanol einsetzt.

30. Verfahren nach den Ansprüchen 27 bis 29, dadurch gekennzeichnet, daß die Reaktionstemperatur 20 bis 80°C beträgt.

31. Verfahren nach Anspruch 27, dadurch gekennzeichnet, daß man ein Amin der Formel II, eine Verbindung der Formel VII

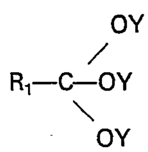

(VII)

(in der $R_1$ und Y die in Anspruch 1 und Anspruch 27 angegebenen Bedeutungen besitzen) und ein Amin der Formel VI in einer einzigen Stufe in Gegenwart eines Katalysators umsetzt.

32. Verfahren nach Anspruch 31, dadurch gekennzeichnet, daß man als Katalysator Schwefelsäure verwendet.

33. Verfahren nach Ansprüchen 31 und 32, dadurch gekennzeichnet, daß die Reaktion bei einer Temperatur von 60 bis 120°C durchgeführt wird.

34. Verfahren zur Herstellung von Verbindungen der allgemeinen Formel I, wie diese in Anspruch 1 oder Anspruch 2 definiert sind, dadurch gekennzeichnet, daß man eine Verbindung der Formel VIII

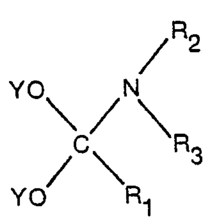

(VIII)

in der $R_1$, $R_2$ und $R_3$ und Y die in Anspruch 1 und in Anspruch 27 angegebenen Bedeutungen besitzen, mit einem Amin der Formel II umsetzt.

35. Verfahren nach Anspruch 34, dadurch gekennzeichnet, daß man die Reaktion bei einer Temperatur von 20 bis 80°C durchführt.

36. Verfahren zur Herstellung von Verbindungen der allgemeinen Formel I, wie sie in Anspruch 1 oder Anspruch 2 definiert sind, dadurch gekennzeichnet, daß man eine Verbindung der Formel IX

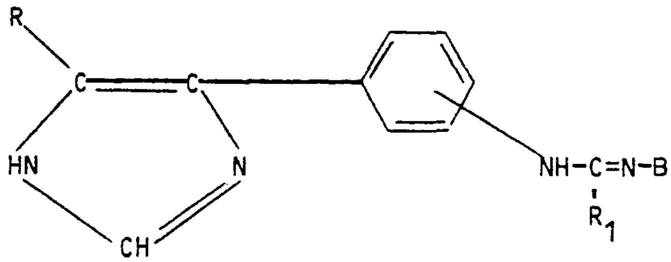

(IX)

in der R und $R_1$ die in Anspruch 1 angegebenen Bedeutungen besitzen und B eine Cyanogruppe, eine Acetylgruppe, eine Carbethoxygruppe oder eine Carbamylgruppe darstellt, mit einem Amin der Formel VI umsetzt.

37. Verfahren nach Anspruch 36, dadurch gekennzeichnet, daß man die Reaktion in Gegenwart von Wasser durchführt.

38. Verfahren nach den Ansprüchen 36 und 37, dadurch gekennzeichnet, daß man die Reaktion bei Raumtemperatur durchführt.

39. Verfahren zur Herstellung von Verbindungen der allgemeinen Formel I, wie sie in Anspruch 1 oder Anspruch 2 definiert sind, dadurch gekennzeichnet, daß man eine Verbindung der Formel XI

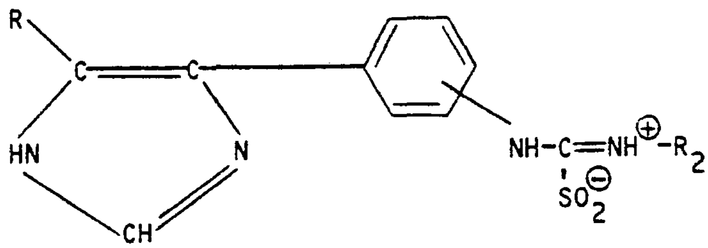

(XI)

in der R und $R_2$ die in Anspruch 1 angegebenen Bedeutungen besitzen, mit einer schwachen organischen Säure umsetzt.

40. Verfahren nach Anspruch 29, dadurch gekennzeichnet, daß man als schwache organische Säure Ameisensäure, Essigsäure oder Propionsäure verwendet.

41. Verfahren zur Herstellung von Verbindungen der allgemeinen Formel I, wie sie in Anspruch 1 oder Anspruch 2 definiert sind, dadurch gekennzeichnet, daß man eine nicht isolierte und in situ durch Umsetzen eines Isonitrils der Formel $C\equiv N-R_2$ mit Imidazol in Gegenwart von AgCl erhaltene Verbindung der Formel XII

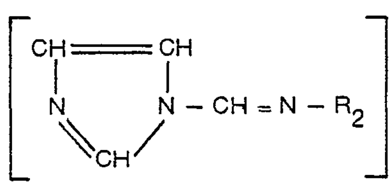

(XII)

in der $R_2$ die in Anspruch 1 angegebenen Bedeutungen besitzt, mit einem Amin der Formel II umsetzt.

42. Verfahren nach Anspruch 41, dadurch gekennzeichnet, daß man ein Amin der Formel II direkt mit dem Isonitril der Formel $C\equiv N-R_2$ in Gegenwart von CuCl umsetzt.

43. Verfahren nach den Ansprüchen 41 und 42, dadurch gekennzeichnet, daß man die Reaktion ohne Lösungsmittel oder in Gegenwart eines inerten organischen Lösungsmittels durchführt.

44. Verfahren nach Anspruch 43, dadurch gekennzeichnet, daß man als inertes organisches Lösungsmittel Ethanol oder Dioxan verwendet.

45. Verfahren nach den Ansprüchen 41 bis 45, dadurch gekennzeichnet, daß die Reaktionstemperatur zwischen 100 und 150°C liegt.

46. Zwischenproduktverbindungen der Formel V

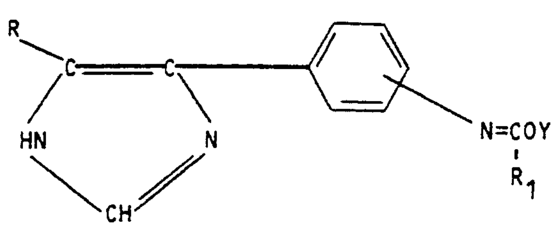

(V)

in der R, $R_1$ und Y die in den Ansprüchen 1 und 27 angegebenen Bedeutungen besitzen, zur Durchführung des Verfahrens gemäß Anspruch 27.

47. Zwischenproduktverbindungen der Formel IX

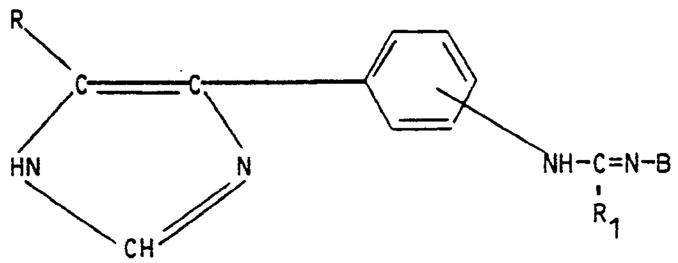

(IX)

in der R, $R_1$ und B die in Anspruch 1 und 36 angegebenen Bedeutungen besitzen, zur Durchführung des Verfahrens gemaß Anspruch 36.

48. Zwischenproduktverbindungen der Formel XI

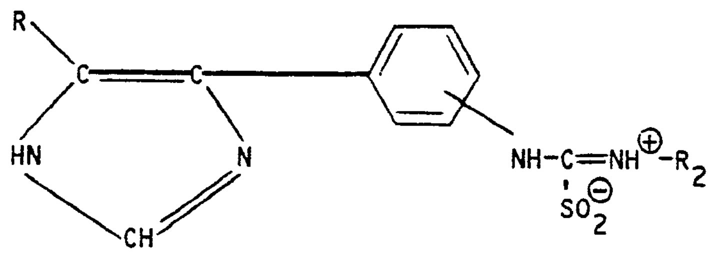

(XI)

in der R und $R_2$ die in Anspruch 1 angegebenen Bedeutungen besitzen, zur Durchführung des Verfahrens gemäß Anspruch 39.

49. Pharmazeutische Zusammensetzungen, die als Wirkstoff mindestens eine Verbindung, wie sie in Anspruch 1 oder Anspruch 2 beansprucht sind, in Kombination mit üblichen pharmazeutischen Trägern oder Hilfsstoffen enthalten.

50. Pharmazeutische Zubereitung nach Anspruch 49 zur Verwendung als Mittel zur Behandlung von Geschwüren.

51. Pharmazeutische Zubereitungen gemäß Anspruch 49 zur Behandlung von Patienten, die an Störungen des Magen-Darm-Traktes leiden.

**Revendications**

1. Composés de formule générale I

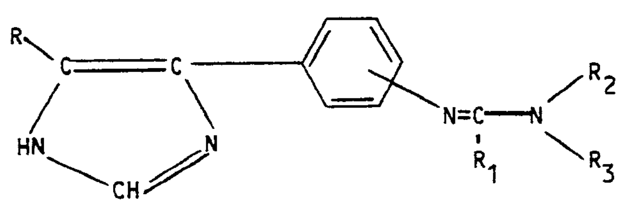

(I)

dans laquelle R, $R_1$ et $R_3$, qui peuvent être identiques ou différents, représentent un atome d'hydrogène ou un groupe alkyle inférieur, et $R_2$ représente un groupe alkyle, alcényle, ou alcynyle linéaire ou ramifié, un groupe cyano, un groupe hydroxyle, un groupe cycloalkyle ou cycloaliphatique-alkyle substitué ou non substitué, un groupe bicyclique, un groupe aralkyle ou aryle (facultativement substitué par des groupes halogène, méthyle, méthoxy ou méthylènedioxy), ou un groupe hétérocyclique-alkyle ou un noyau héterocyclique insaturé à 5 chaînons, substitué ou non substitué, et qui peut également contenir un autre hétéroatome.

2. Sels d'addition d'acides non toxiques des composés de formule générale (I) tels que revendiqués dans la revendication 1.

3. Sels tels que revendiqué dans la revendication 2, formés avec l'acide chlorhydrique, fumarique, méthanesulfonique ou maléique.

4. Composés tels que revendiqués dans la revendication 1, dans lesquels R, $R_1$ et $R_3$ représentent un atome d'hydrogène.

5. Composés tels que revendiqués dans les revendications 1 et 4, dans lesquels le radical amidine est en position para du noyau benzénique.

6. Composés tel que revendiqués dans les revendications 1, 4 et 5, dans lesquels $R_2$ représente un groupe hydroxyle, méthyle, éthyle, n-propyle, isopropyle, n-butyle, isobutyle, sec-butyle, t-butyle, néopentyle, hexyle, heptyle, octyle, allyle, α-méthylallyle, prényle, propargyle, α-méthylpropargyle, cyano, norbornyle, benzyle, cyclopropyle, cyclopropylméthyle, diméthylcyclopropylméthyle, menthyle, cyclohexyle, cyclohexylméthyle, phényle, p-chlorophényle, p-méthylphényle, p-méthoxyphényle, méthylènedioxyphényle ou 2-furylméthyle.

7. Sels d'addition d'acides non toxiques des composés tels que revendiqués dans la revendication 6.

8. Sels tel que revendiqués dans la revendication 7, formés avec l'acide chlorhydrique, fumarique, méthanesulfonique ou maléique.

9. La N-éthyl-N'-[4-(imidazole-4-yl)-phényl]-formamidine.

10. La N-isopropyl-N'-[4-(imidazole-4-yl)-phényl]-formamidine.

11. La N-allyl-N'-[4-(imidazole-4-yl)-phényl]-formamidine.

12. La N-n-propyl-N'-[4-(imidazole-4-yl)-phényl]-formamidine.

13. La N-sec-butyl-N'-[4-(imidazole-4-yl)-phényl]-formamidine.

14. La N-isobutyl-N'-[4-(imidazole-4-yl)-phényl]-formamidine.

15. La N-prényl-N'-[4-(imidazole-4-yl)-phényl]-formamidine.

16. La N-cyclopropylméthyl-N'-[4-(imidazole-4-yl)-phényl]-formamidine.

17. La N-cyclopropyl-N'-[4-(imidazole-4-yl)-phényl]-formamidine.

18. La N-(α-méthylallyl)-N'-[4-(imidazole-4-yl)-phényl]-formamidine.

19. La N-néopentyl-N'-[4-(imidazole-4-yl)-phényl]-formamidine.

20. La N-(2,2-diméthyl)-cyclopropylméthyl-N'-[4-(imidazole-4-yl)-phényl]-formamidine.

21. Composés tels que revendiqués dans l'une quelconque des revendications 9 à 20 sous la forme de leurs sels d'addition de l'acide chlorhydrique, fumarique, méthanesulfonique ou maléique.

22. Un procédé pour la préparation des composés de formule générale I, tels que définis dans la revendication 1 ou la revendication 2, qui consiste à faire réagir un composé de la formule II

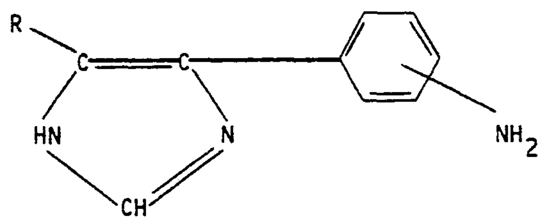

(II)

(dans laquelle R est tel que défini dans la revendication 1) avec un composé de formule III

$$\begin{matrix} R_1 \\ \diagdown \\ C{=}NH^{\oplus}{-}R_2\,X^{\ominus} \\ \diagup \\ A \end{matrix} \qquad (III)$$

dans laquelle $R_1$ et $R_2$ sont tels que définis dans la revendication 1, $X^{\ominus}$ représente l'anion d'un chlorhydrate ou d'un fluoroborate et A représente un groupe benzoyloxy, du chlore ou un groupe éthoxy.

23. Un procéde tel que revendiqué dans la revendication 22, dans lequel on fait réagir un composé de formule III sous forme de base.

24. Un procédé tel que revendiqué dans les revendications 22 et 23, dans lequel la réaction est mise en oeuvre en présence d'un solvant organique inerte.

25. Un procédé tel que revendiqué dans la revendication 24, dans lequel le solvant organique inerte consiste en éthanol, dioxanne ou acétone.

26. Un procédé tel que revendiqué dans les revendications 22 à 25, dans lequel la réaction est mise en oeuvre à une température de 20 à 60°C.

27. Un procédé pour la préparation de composés de formule générale I tels que définis dans la revendication 1 ou la revendication 2, qui consiste à faire réagir un composé de formule V

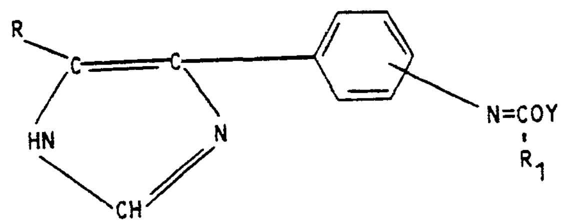

(V)

dans laquelle R et $R_1$ sont tels que définis dans la revendication 1, et Y représente un groupe méthyle ou éthyle, avec une amine de formule VI

$$H_2N—R_2 \qquad (VI)$$

où $R_2$ est tel que défini dans la revendication 1.

28. Un procédé tel que revendiqué dans la revendication 27, dans lequel la réaction est mise en oeuvre dans un solvant organique inerte.

29. Un procédé tel que revendiqué dans la revendication 28, dans lequel le solvant organique inerte est l'éthanol.

30. Un procédé tel que revendiqué dans les revendications 27 à 29, dans lequel la température de réaction est de 20 à 80°C.

31. Un procédé tel que revendiqué dans la revendication 27, dans lequel une amine de formule II, un composé de formule VII

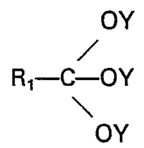

(VII)

(dans laquelle $R_1$ et Y sont tels que définis dans la revendication 1 et la revendication 27) et une amine de formule VI sont mis à réagir en un seul stade en présence d'un catalyseur.

32. Un procédé tel que revendiqué dans la revendication 31, dans lequel le catalyseur est l'acide sulfurique.

33. Un procédé tel que revendiqué dans les revendications 31 et 32, dans lequel le réaction est mise en oeuvre à une température de 60 à 120°C.

34. Un procédé pour la préparation de composés de formule générale I, tels que revendiqués dans la revendication 1 ou la revendication 2, qui consiste à faire réagir un composé de formule VIII

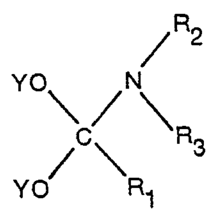

(VIII)

dans laquelle $R_1$, $R_2$ et $R_3$ et Y sont tels que définis dans la revendication 1, et la revendication 27, avec une amine de formule II.

35. Un procédé revendiqué dans la revendication 34, dans lequel la réaction est mise en oeuvre à une température de 20 à 80°C.

36. Un procédé pour la préparation de composés de formule générale I, tels que revendiqués dans la revendication 1 ou la revendication 2, qui consiste à faire réagir un composé de formule IX

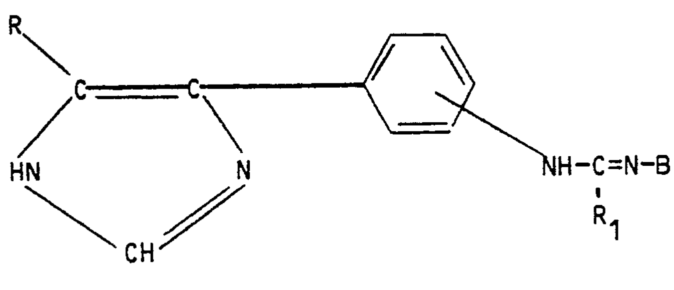

(IX)

dans laquelle R et $R_1$ sont tels que définis dans la revendication 1, B représente un groupe cyano, acétyle, carbéthoxy ou carbamyle, avec une amine de formule VI.

37. Un procédé tel que revendiqué dans la revendication 36, dans lequel la réaction est réalisée en présence d'eau.

38. Un procédé tel que revendiqué dans les revendications 36 et 37, dans leuqel la réaction est réalisée à la température ambiante.

39. Un procédé pour la préparation de composé de formule générale I, tels que revendiqués dans la revendication 1 ou la revendication 2, qui consiste à faire réagir un composé de formule XI

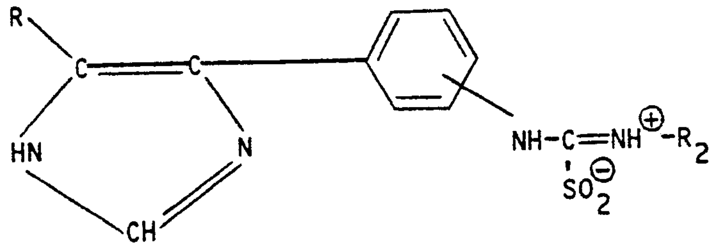

(XI)

dans laquelle R et $R_2$ sont définis dans la revendication 1, avec un acide organique faible.

40. Un procédé tel que revendiqué dans la revendication 39, dans lequel l'acide organique faible est constitué par l'acide formique, acétique ou propionique.

41. Un procédé pour la préparation de composés de formule générale I, tels que revendiqués dans la revendication 1 ou la revendication 2, qui consiste à faire réagir un composé non isolé, obtenu in situ entre l'isonitrile de formule C≡N—$R_2$ et l'imidazole en présente de AgCl, de formule XII

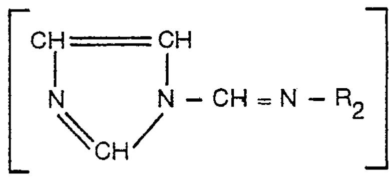

(XII)

dans laquelle $R_2$ est tel que défini dans la revendication 1, avec une amine de formule II.

42. Un procédé tel que revendiqué dans la revendicaion 41, dans lequel une amine de formule II est mise directement à réagir avec l'isonitrile de formule C≡N—$R_2$ en présence de CuCl.

43. Un procédé tel que revendiqué dans les revendications 41 et 42, dans lequel la réaction est mise en oeuvre sans solvant ou en présence d'un solvant organique.

44. Un procédé tel que revendiqué dans les revendication 43, dans lequel le solvant organique inerte est constitué par l'éthanol ou le dioxanne.

45. Un procédé tel que revendiqué dans les revendications 41 à 44, dans lequel la température réactionnelle est entre 100 et 150°C.

46. Composés intermédiaires de formule

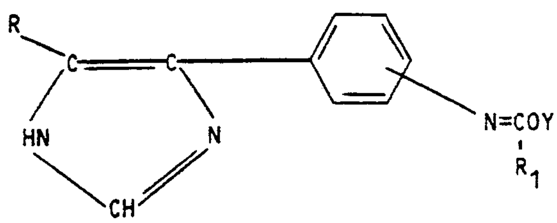

(V)

dans laquelle R, $R_1$ et Y sont tels que définis dans les revendications 1 et 27 pour la mise en oeuvre du procédé tel que revendiqué dans la revendication 27.

47. Composés intermédiaires de formule

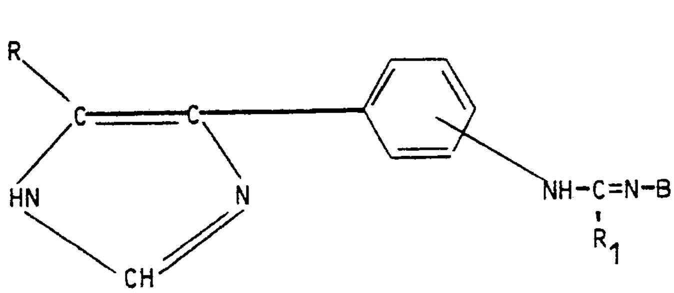

(IX)

dans laquelle R, $R_1$ et B sont tels que définis dans la revendication 1 et la revendication 36, pour la mise en ouevre du procédé tel que revendiqué dans la revendication 36.

48. Composé intermédiaires de formule

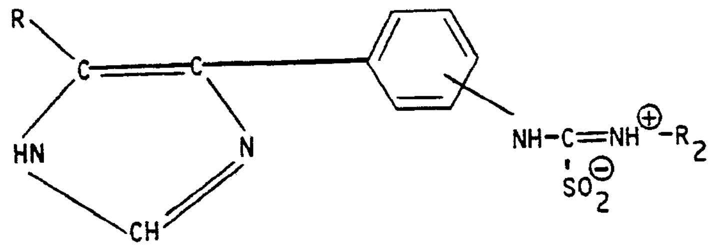

(XI)

dans laquelle R et $R_2$ sont tels que définis dans la revendication 1, pour la mise en oeuvre du procédé tel que revendiqué dans la revendication 39.

49. Compositions pharmaceutiques comportant comme ingrédient actif au moins un composé tel que revendiqué dans la revendication 1 ou la revendication 2 en association avec des supports ou excipients pharmaceutiques habituels.

50. Composition pharmaceutique telle que revendiquée dans la revendication 49 pour utilisation en tant qu'anti-ulcères.

51. Composition pharmaceutiques telles que revendiquées dans la revendication 49 pour le traitement de patients souffrant de troubles de voies gastro-intestinales.